# EUROPEAN PATENT SPECIFICATION

(11) **EP 3 863 695 B1**
(45) Date of publication and mention of the grant of the patent: **03.05.2023**
(21) Application number: 19797801.8
(22) Date of filing: 09.10.2019
(51) Int. Cl.: A61M 1/16, A61M 1/36

(54) **APPARATUS FOR THE MEASUREMENT OF CARBON DIOXIDE IN A WORKING GAS**
VORRICHTUNG ZUR MESSUNG VON KOHLENDIOXID IN EINEM ARBEITSGAS
APPAREIL DE MESURE DE DIOXYDE DE CARBONE DANS UN GAZ DE TRAVAIL

(30) Priority: 10.10.2018 IT 201800009331
(43) Date of publication of application: 18.08.2021
(73) Proprietor: Eurosets S.r.l., 41036 Medolla (MO) (IT)
(72) Inventor: GHELLI, Nicola, 41036 Medolla (MO) (IT); FONTANILI, Paolo, 41036 Medolla (MO) (IT)
(74) Representative: Zoli, Filippo
(86) International application number: PCT/IB2019/058603
(87) International publication number: WO 2020/075087

(56) References cited:
- EP-B1- 2 841 126
- WO-A1-2011/053414
- WO-A1-2018/026671

## Description

### Technical Field

The present invention relates to an apparatus for the measurement of carbon dioxide in a working gas.

### Background Art

One of the possible medical applications of this type of system is inside extracorporeal blood circulation circuits.

In fact, it is known that during particular heart surgery, the establishment is required of extracorporeal blood circulation the main purpose of which is the perfusion of vital organs, i.e., the vascularization of the same with oxygenated blood, to ensure their proper functioning.

In particular, known types of systems are equipped with an oxygenation device inside which the blood taken from the venous line of the patient is enriched with oxygen before being introduced into the arterial line of the patient. Appropriately, the oxygenation device comprises an inlet channel and an outlet channel of a working gas intended to supply oxygen to the blood and/or to remove carbon dioxide from it.

The amount of carbon dioxide removed by the oxygenator is therefore a parameter of fundamental importance to monitor the progress of extracorporeal circulation and to assess the correct oxygenation of the blood.

To this end, or to detect the amount of carbon dioxide removed from the blood coming from the patient, known types of systems generally comprise measuring devices defined, in jargon, "capnometers", which are connected in a fluid-operated manner to the oxygenation device through a suitable connecting line, the purpose of which is to convey the working gas coming out from the oxygenation device into the measuring device.

In particular, known types of measuring devices are provided with an inlet for the working gas, with an emitter of an optical signal adapted to cross the working gas entering the measuring device through the inlet, and with a receiver placed opposite the emitter and adapted to receive the optical signal emitted by the latter.

This way, through the analysis of the optical signal received by the receiver, e.g., through spectrophotometry for the absorption of the optical signal, it is possible to trace the amount of carbon dioxide contained in the working gas. This type of system has however a number of drawbacks related to the risk of compromising the optical signal and/or altering the reception thereof.

In fact, it often occurs that at least part of the working gas coming out of the oxygenation device condensates inside the connecting line and, by depositing at the point where the transmitter and/or the receiver is located, compromises the optical signal and/or alters the reception thereof.

Consequently, the analysis of the optical signal received by the receiver under these conditions makes the measurement of the level of carbon dioxide present in the working gas inaccurate and, in some cases, unreliable.

Some devices for the measurement of the amount of carbon dioxide contained in the working gas used in blood oxygenation devices in an extracorporeal environment are known from WO 2011/053414 A1, EP 2841126 A1, WO 2018/026671 A1.

These devices of known type are provided with auxiliary means adapted to regulate the pressure along the relevant circuits in order to facilitate the runoff of the condensate generated during the flow of the working gas inside the relative ducts.

The device known from WO 2011/053414 A1 has, e.g., a vacuum pump located downstream of the blood oxygenation device.

The device known from EP 2841126 A1 comprises a control unit adapted to generate pressure peaks which allow the removal of the condensate which forms as a result of the flow of the working gas coming out of the oxygenation device. These devices of known type do also have a number of drawbacks, as they are constructively complex and necessarily require the use of auxiliary means to exert an active pressure on the working gas, besides being very costly.

Moreover, these devices of known type are not very reliable because, being made up of a large number of components, the malfunction of one of them can affect the correct reading of the amount of carbon dioxide, as well as the proper treatment of blood during extracorporeal circulation.

### Description of the Invention

The main aim of the present invention is to devise an apparatus which permits measuring, in an effective, precise and reliable way, the level of carbon dioxide present in a working gas, particularly in the working gas which flows through a blood oxygenation device.

Within this aim, one of the objects of the present invention is to allow the easy and safe removal of the condensate which is generated during the transfer of the working gas from the outlet of the oxygenation device to the relevant measuring device.

Another object of the present invention is to devise an apparatus for the measurement of carbon dioxide in a working gas which allows overcoming the aforementioned drawbacks of the prior art in the context of a simple, rational, easy, effective to use and affordable solution.

The aforementioned objects are achieved by the present apparatus for the measurement of carbon dioxide in a working gas according to claim 1.

### Brief Description of the Drawings

Other characteristics and advantages of the present invention will be more evident from the description of several preferred, but not exclusive, embodiments of an apparatus for the measurement of carbon dioxide in a working gas illustrated by way of an example, but not limited thereto, in the accompanying drawings, wherein:
Figure 1 is a schematic view of an embodiment of the apparatus according to the invention;
Figure 2 is a schematic view of an alternative embodiment of the apparatus according to the invention.

### Embodiments of the Invention

With particular reference to these illustrations, reference numeral 1 globally designates an apparatus for the measurement of carbon dioxide in a working gas.

The apparatus 1 for the measurement of carbon dioxide in a working gas comprises:
- at least one oxygenation device 2 of an extracorporeal blood flow provided with at least one entry 3 and one outlet 4 for a working gas 16 used to supply oxygen and/or to remove CO₂ from the blood flow;
- at least one measuring device 5 provided with at least one inlet 6 of the working gas 16 coming out of the oxygenation device 2 and configured to measure the level of carbon dioxide present inside the working gas 16;
- at least one connecting line 7 for the connection of the outlet 4 to the inlet 6 adapted to convey the working gas 16 coming out of the oxygenation device 2 entering the measuring device 5.

The term "oxygenation device" means any medical device used for the oxygenation and/or the removal of CO2 from a blood stream by means of a working gas.

The measuring device 5, on the other hand, is preferably of the type of a "capnometer", e.g. with absorption spectrophotometry, with which it is possible to identify the level of carbon dioxide present inside the working gas 16 by analyzing a light beam, generally in the infrared spectrum, which crosses the working gas 16 coming from the oxygenation device 2.

Preferably, the connecting line 7 is of the type of a hollow body of substantially tubular shape for the containment and channeling of fluids.

The apparatus 1 comprises outflow means 8 for the condensate 15 formed by the working gas 16 along the connecting line 7 outside the same.

More in particular, the outflow means 8 are adapted to remove, by conveying it outwards, the condensate which forms as a result of the contact of the working gas 16, which comes out of the outlet 4, with the internal walls of the connecting line 7.

According to the invention, the outflow means 8 comprise at least a branch line 9 communicating on one side with the connecting line 7 and on the opposite side with the external environment. The branch line 9 is therefore at room pressure.

In other words, the branch line 9 is in fluid-operated connection with the connecting line 7 and communicates, on the opposite side, with the external environment, where it releases the condensate 15 formed by the flow of working gas 16 along the connecting line 7.

The apparatus 1 is therefore of the passive type, i.e. it has no auxiliary means, such as pumping means, heating means or the like, arranged along at least one of either the connecting line 7 or the branch line 9 and adapted to exert a pressure greater or less than zero inside them to allow the condensate which forms as a result of the flow of the working gas 16 to come out.

The branch line 9 is placed at a lower level than the outlet 4 of the working gas from the oxygenation device 2 and with respect to the inlet 6 of the working gas into the measuring device 5.

As described for the connecting line 7, the branch line 9 is preferably of the type of a hollow body with a substantially tubular shape for the containment and channeling of fluids.

In particular, the branch line 9 comprises at least one outflow length 10 of the working gas 16 outside the connecting line 7, which comprises at least a first and at least a second passage length 13, 14 of the working gas 16 arranged upstream and downstream, respectively, of the outflow length 10 with respect to the direction of forward movement of the working gas itself towards the measuring device 5.

The outflow length 10 is oriented downwards and is positioned at a lower level with respect to the passage lengths 13 and 14.

Appropriately, the cross-section of the first passage length 13, of the second passage length 14 and of the outflow length 10 are of the calibrated type, i.e. their size is defined in such a way as to facilitate the outflow of the condensate outwards.

Preferably, the first passage length 13 has a cross-section for the passage of the working gas 16 which is greater than or equal to the cross-section for the passage of the outflow length 10.

In an alternative embodiment, not shown in the figures, however, the first passage length 13 has a cross-section which is smaller than or equal to the cross-section for the passage of the outflow length 10.

In both embodiments, the second passage length 14 has a cross-section for the passage of the working gas 16 which is greater than or equal to the cross-section for the passage of the outflow length 10.

Such solution enables the branch line 9 to extract the condensate 15 formed by the working gas 16 inside the connecting line 7 without compromising the operation of the measuring device 5.

In fact, the outflow length 10 also enables a variable quantity of working gas 16 to leave the connecting line 7 together with the condensate 15, so its passage cross-section must be such as to allow the amount of working gas 16 which reaches the measuring device 5 to be sufficient to perform an accurate measurement of the level of carbon dioxide present in the same.

With reference to the embodiments of the apparatus 1 shown in the figures, the first passage length and the second passage length are of circular conformation. Alternative embodiments of the connecting line 7 and of the branch line 9 cannot however be ruled out wherein the first and the second passage lengths are of different conformation, e.g. of oval, square or similar shape.

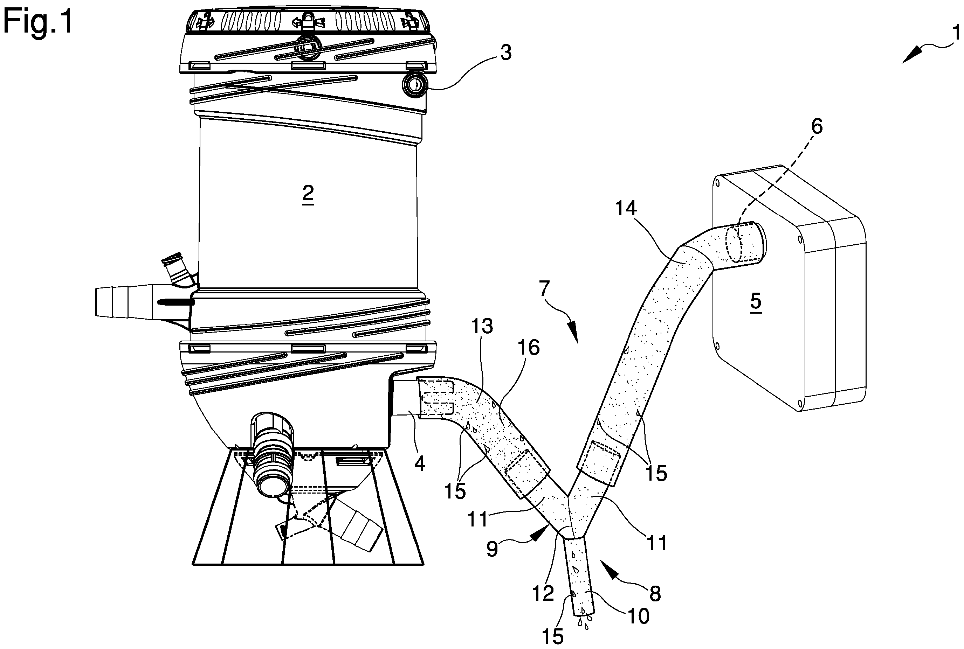

A possible embodiment of the apparatus 1 is shown in Figure 1, wherein the branch line 9 is connected in a removable manner to the connecting line 7.

In particular, the branch line 9 comprises at least one pair of connecting lengths 11 which are connected in a fluid-operated manner to each other and to the outflow length 10 at a single joining point 12.

Furthermore, the first passage length 13 is connected in a fluid-operated and removable manner to the outlet 4 and to one of the connecting lengths 11 and the second passage length 14 is connected in a fluid-operated and removable manner to the inlet 6 and to the other of the connecting lengths 11.

Preferably, the branch line 9 comprises at least a first extremal portion made on each of the connecting lengths 11, and the connecting line 7 comprises at least a second extremal portion made on each of the passage lengths 13, 14 and associable in a removable and interlocking manner with the first extremal portion.

In particular, the first extremal portion and the second extremal portion have cross-sections for the passage of the working gas 16 of different dimensions respectively to enable the interlocking of one inside the other.

In other words, one of either the first extremal portion and the second extremal portion is fitted in a removable interlocked manner into the other of either the first extremal portion or the second extremal portion.

This way, the connecting lengths 11 and the passage lengths 13, 14 ensure fluid-operated continuity between the connecting line 7 and the branch line 9.

In particular, with reference to the embodiment of the apparatus 1 shown in Figure 1, the first extremal portion and the second extremal portion are made in a single body piece with the branch line 9 and with the connecting line 7 respectively.

In fact, the cross-section for the passage of the first extremal portion and the cross-section for the passage of the second extremal portion have a substantially circular conformation.

Moreover, the cross-section for the passage of the first extremal portion is smaller in size than the cross-section for the passage of the second extremal portion, in order to allow associating by interlocking at least part of the first extremal portion into at least part of the second extremal portion, abutting the outer surface of the first portion with the inner surface of the second extremal portion.

Alternative embodiments of the branch line 9 and of the connecting line 7 cannot however be ruled out, wherein the cross-section for the passage of the first extremal portion is larger in size than the cross-section for the passage of the second extremal portion, thereby making it possible, contrary to what has been described above, to associate by interlocking at least part of the second extremal portion into at least part of the first extremal portion. Advantageously, the branch line 9 has a substantially Y-shaped conformation, as shown in Figure 1.

Alternative embodiments of the outflow means 8 wherein the branch line 9 has a different shape, e.g. a "T" shape, cannot be ruled out.

In particular, the connecting lengths 11 are arranged substantially converging towards the outflow length 10, which faces the resting surface onto which the oxygenation device 2 and the measuring device 5 rest.

This way, the condensate 15 formed by the working gas 16 inside the connecting line 7 is facilitated to escape through the outflow length 10 thanks to the slope defined by the connecting lengths 11.

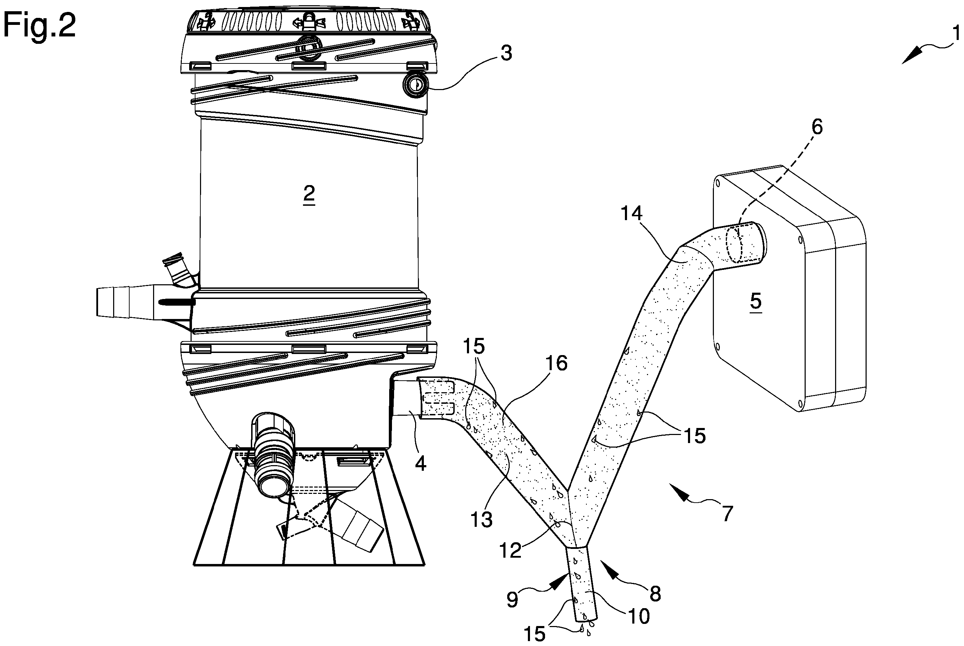

In the alternative embodiment of the apparatus 1 shown in Figure 2, the connecting line 7 and the branch line 9 are made in a single body piece.

In fact, in this embodiment, the branch line 9 comprises only the outflow length 10, which then branches off directly from the connecting line 7.

More particularly, the first passage length 13 and the second passage length 14 of the connecting line 7 converge downwards and the outflow length 10 is connected in a fluid-operated manner to these passage lengths 13, 14 at the joining point 12 thereof.

In this embodiment the connecting lengths 11 substantially coincide with the passage lengths 13 and 14.

This way, the connecting line 7 and the branch line 9 make a single communication channel which can be connected in a fluid-operated manner to the outlet 4 and to the inlet 6 and which allows expelling the condensate formed by the working gas.

Preferably, also in this embodiment the connecting line 7, made in a single body piece with the branch line 9, has a substantially Y-shaped conformation, as shown in Figure 2.

Alternative embodiments of the outflow means 8 cannot however be ruled out, wherein the connecting line 7 formed in a single body piece with the branch line 9 has a different shape, for example a "T" shape.

The operation of the apparatus 1 is as follows.

Initially, it is necessary to connect the outlet 4 and the inlet 6 to the lengths of the branch line 9 by means of the passage lengths 13, 14, so as to connect the oxygenation device 2 to the measuring device 5 in a fluid-operated manner.

In the embodiment shown in Figure 1, the branch line 9 is connected to the connecting line 7 by means of the relevant connecting lengths 11, which engage in a removable way with the passage lengths 13, 14.

Furthermore, it is advantageous to orient the outflow length 10 turned substantially downwards in order to facilitate the expulsion of the condensate 15.

This way, during the process of oxygenating the extracorporeal blood flow, the condensate 15 formed by the working gas 16 which crosses the connecting line 7 is naturally channeled towards the outflow length 10, which, in turn, channels the condensate 15 outside the connecting line 7.

In particular, as described with reference to the previous embodiment, the passage lengths 13, 14 are positioned substantially converging towards the outflow length 10, which is turned downwards, e.g. towards the supporting surface on which the oxygenation device 2 and the measuring device 5 rest. The operation of the embodiment of Figure 2 is completely identical to that described with reference to the embodiment of Figure 1.

In this case, the connection of the branch line 9 to the connecting line 7 is easier because they are made in a single body piece.

In fact, as described above, it is initially necessary to connect the outlet 4 and the inlet 6 to the passage lengths 13, 14, so as to connect the oxygenation device 2 to the measuring device 5 in a fluid-operated manner.

In this case too, it is a good idea to orient the outflow length 10 substantially downwards in order to facilitate the expulsion of the condensate 15.

It has in practice been ascertained that the described invention achieves the intended objects.

In particular, the fact is underlined that the branch channel allows the condensate formed by the working gas inside the connecting line to come out of the latter, enabling the measuring device to effectively, accurately and reliably detect the amount of carbon dioxide removed from the blood.

The removal of the condensate that forms as a result of the passage of the working gas inside the connecting line takes place without the use of auxiliary means adapted to adjust the pressure along the connecting line but simply according to the size of the passage lengths of the working gas and of the position of the outflow length.

The apparatus forming the subject of the present invention is therefore significantly simpler from the construction point of view than the devices of known type and more reliable in terms of operation.

## Claims

1. Apparatus (1) for the measurement of carbon dioxide in a working gas comprising:
- at least one oxygenation device (2) of an extracorporeal blood flow provided with at least one entry (3) and one outlet (4) for a working gas (16) used to supply oxygen and/or to remove CO2 from the blood flow;
- at least one measuring device (5) provided with at least one inlet (6) of the working gas (16) coming out of said oxygenation device (2) and configured to measure the level of carbon dioxide present inside the working gas (16);
- at least one connecting line (7) for the connection of said outlet (4) to said inlet (6) adapted to convey the working gas (16) coming out of said oxygenation device (2) entering said measuring device (5);
- outflow means (8) of a condensate (15) formed by the working gas (16) along the connecting line (7) on the outside thereof;
**characterized by** the fact that said outflow means (8) comprise at least one branch line (9) communicating on one side with said connecting line (7) and on the opposite side with the external environment, said branch line (9) being at room pressure, wherein said branch line (9) is placed at a lower level than said outlet (4) of the oxygenation device (2) and with respect to said inlet (6) of the measuring device (5).

2. Apparatus (1) according to claim 1, **characterized by** the fact that it has no auxiliary means arranged along at least one of either said connecting line (7) or said branch line (9) and adapted to exert a pressure greater or less than zero inside them.

3. Apparatus (1) according to one or more of the preceding claims, **characterized by** the fact that said branch line (9) comprises at least one outflow length (10) of the working gas (16) outside said connecting line (7), the latter comprising at least a first and at least a second passage length (13, 14) of the working gas (16) arranged upstream and downstream, respectively, of said outflow length (10) with respect to the direction of forward movement of the working gas itself towards said measuring device (5).

4. Apparatus (1) according to claim 3, **characterized by** the fact that the cross-sections of said first passage length (13), of said second passage length (14) and of said outflow length (10) are of the calibrated type.

5. Apparatus (1) according to claim 4, **characterized by** the fact that said first passage length (13) has a cross-section for the passage of the working gas (16) which is greater than or equal to the cross-section for the passage of said outflow length (10).

6. Apparatus (1) according to one or more of claims 3 to 5, **characterized by** the fact that said second passage length (14) has a cross-section for the passage of the working gas (16) which is greater than or equal to the cross-section for the passage of said outflow length (10).

7. Apparatus (1) according to one or more of the preceding claims, **characterized by** the fact that said branch line (9) is connectable in a removable manner to said connecting line (7).

8. Apparatus (1) according to one or more of claims 3 to 7, **characterized by** the fact that said branch line (9) comprises at least one pair of connecting lengths (11) which are connected in a fluid-operated manner to each other and to said outflow length (10) at a single joining point (12), and by the fact that said first passage length (13) is connected in a fluid-operated and removable manner to said outlet (4) and to one of said connecting lengths (11) and said second passage length (14) is connected in a fluid-operated and removable manner to said inlet (6) and to the other of said connecting lengths (11).

9. Apparatus (1) according to claim 8, **characterized by** the fact that said connecting lengths (11) are arranged substantially converging towards said outflow length (10).

10. Apparatus (1) according to claim 8 or 9, **characterized by** the fact that said branch line (9) has a "Y" conformation.

11. Apparatus (1) according to one or more of the preceding claims, **characterized by** the fact that said connecting line (7) and said branch line (9) are made in a single body piece.

## Patentansprüche

1. Vorrichtung (1) zur Messung von Kohlendioxid in einem Arbeitsgas, aufweisend:
- mindestens eine Oxygenierungsvorrichtung (2) eines extrakorporalen Blutflusses, die mindestens einen Eingang (3) und einen Auslass (4) für ein Arbeitsgas (16) aufweist, das zur Zufuhr von Sauerstoff und/oder zur Entfernung von CO2 aus dem Blutfluss dient;
- mindestens eine Messvorrichtung (5), die mindestens einen Einlass (6) für das aus der Oxygenierungsvorrichtung (2) austretende Arbeitsgas (16) aufweist und dazu ausgebildet ist, den im Arbeitsgas (16) vorliegenden Kohlendioxidgehalt zu messen;
- mindestens eine Verbindungsleitung (7) für die Verbindung des Auslasses (4) mit dem Einlass (6), die dazu ausgebildet ist, das aus der Oxygenierungsvorrichtung (2) austretende Arbeitsgas (16) in die Messvorrichtung (5) zu leiten;
- Ausflussmittel (8) für ein Kondensat (15), das von dem Arbeitsgas (16) entlang der Verbindungsleitung (7) an der Außenseite derselben gebildet wird;
**dadurch gekennzeichnet, dass** die Ausflussmittel (8) mindestens eine Verzweigungsleitung (9) aufweisen, die auf einer Seite mit der Verbindungsleitung (7) und auf der gegenüberliegenden Seite mit der äußeren Umgebung in Verbindung steht, wobei die Verzweigungsleitung (9) unter Umgebungsdruck steht, wobei die Verzweigungsleitung (9) auf einer niedrigeren Höhe als der Auslass (4) der Oxygenierungsvorrichtung (2) und in Bezug auf den Einlass (6) der Messvorrichtung (5) angeordnet ist.

2. Vorrichtung (1) nach Anspruch 1, **dadurch gekennzeichnet, dass** sie keine Hilfsmittel aufweist, die entlang der Verbindungsleitung (7) und/oder der Verzweigungsleitung (9) angeordnet sind und die ausgebildet sind, um in ihnen einen Druck größer oder kleiner als Null zu bewirken.

3. Vorrichtung (1) nach einem oder mehreren der vorhergehenden Ansprüche, **dadurch gekennzeichnet, dass** die Verzweigungsleitung (9) mindestens eine Ausströmlänge (10) des Arbeitsgases (16) außerhalb der Verbindungsleitung (7) aufweist, wobei letztere mindestens eine erste und mindestens eine zweite Durchgangslänge (13, 14) des Arbeitsgases (16) aufweist, die stromauf bzw. stromab der Ausströmlänge (10) in Bezug auf die Richtung der Vorwärtsbewegung des Arbeitsgases selbst zur Messvorrichtung (5) hin angeordnet sind.

4. Vorrichtung (1) nach Anspruch 3, **dadurch gekennzeichnet, dass** die Querschnitte der ersten Durchgangslänge (13), der zweiten Durchgangslänge (14) und der Ausströmlänge (10) vom kalibrierten Typ sind.

5. Vorrichtung (1) nach Anspruch 4, **dadurch gekennzeichnet, dass** die erste Durchgangslänge (13) einen Querschnitt für den Durchgang des Arbeitsgases (16) aufweist, der größer als der oder gleich dem Querschnitt für den Durchgang der Ausströmlänge (10) ist.

6. Vorrichtung (1) nach einem oder mehreren der Ansprüche 3 bis 5, **dadurch gekennzeichnet, dass** die zweite Durchgangslänge (14) einen Querschnitt für den Durchgang des Arbeitsgases (16) aufweist, der größer als der oder gleich dem Querschnitt für den Durchgang der Ausströmlänge (10) ist.

7. Vorrichtung (1) nach einem oder mehreren der vorhergehenden Ansprüche, **dadurch gekennzeichnet, dass** die Verzweigungsleitung (9) lösbar mit der Verbindungsleitung (7) verbindbar ist.

8. Vorrichtung (1) nach einem oder mehreren der Ansprüche 3 bis 7, **dadurch gekennzeichnet, dass** die Verzweigungsleitung (9) mindestens ein Paar von Verbindungslängen (11) aufweist, die an einer einzigen Zusammenführungsstelle (12) miteinander und mit der Ausströmlänge (10) fluidisch verbunden sind, und dadurch, dass die erste Durchgangslänge (13) mit dem Auslass (4) und mit einer der Verbindungslängen (11) und die zweite Durchgangslänge (14) mit dem Einlass (6) und mit der anderen der Verbindungslängen (11) fluidisch und lösbar verbunden ist.

9. Vorrichtung (1) nach Anspruch 8, **dadurch gekennzeichnet, dass** die Verbindungslängen (11) im Wesentlichen konvergierend zu der Ausströmlänge (10) angeordnet sind.

10. Vorrichtung (1) nach Anspruch 8 oder 9, **dadurch gekennzeichnet, dass** die Verzweigungsleitung (9) eine "Y"-Form aufweist.

11. Vorrichtung (1) nach einem oder mehreren der vorhergehenden Ansprüche, **dadurch gekennzeichnet, dass** die Verbindungsleitung (7) und die Verzweigungsleitung (9) in einem einzigen Bauteil ausgeführt sind.

## Revendications

1. - Appareil (1) pour la mesure de dioxyde de carbone dans un gaz de travail, comprenant :
- au moins un dispositif d'oxygénation (2) d'un écoulement sanguin extracorporel, comportant au moins une entrée (3) et une sortie (4) pour un gaz de travail (16), utilisé pour introduire de l'oxygène et/ou pour retirer du CO2 à partir de l'écoulement sanguin ;
- au moins un dispositif de mesure (5) comportant au moins une entrée (6) du gaz de travail (16) sortant dudit dispositif d'oxygénation (2) et configuré pour mesurer le niveau de dioxyde de carbone présent à l'intérieur du gaz de travail (16) ;
- au moins un conduit de liaison (7) pour la liaison de ladite sortie (4) à ladite entrée (6), apte à transporter le gaz de travail (16) sortant dudit dispositif d'oxygénation (2) entrant dans ledit dispositif de mesure (5) ;
- des moyens d'écoulement de sortie (8) d'un condensat (15) formé par le gaz de travail (16) le long du conduit de liaison (7) sur son côté extérieur ;
**caractérisé par le fait que** lesdits moyens d'écoulement de sortie (8) comprennent au moins un conduit de dérivation (9) communiquant d'un côté avec ledit conduit de liaison (7) et du côté opposé avec l'environnement externe, ledit conduit de dérivation (9) se trouvant à pression ambiante, ledit conduit de dérivation (9) étant placé à un niveau inférieur à ladite sortie (4) du dispositif d'oxygénation (2) et par rapport à ladite entrée (6) du dispositif de mesure (5).

2. - Appareil (1) selon la revendication 1, **caractérisé par le fait qu'**il n'a pas de moyens auxiliaires agencés le long d'au moins l'un dudit conduit de liaison (7) ou dudit conduit de dérivation (9) et conçus pour exercer une pression supérieure ou inférieure à zéro à l'intérieur d'eux.

3. - Appareil (1) selon une ou plusieurs des revendications précédentes, **caractérisé par le fait que** ledit conduit de dérivation (9) comprend au moins un tronçon d'écoulement de sortie (10) du gaz de travail (16) à l'extérieur dudit conduit de liaison (7), ce dernier comprenant au moins un premier et au moins un second tronçon de passage (13, 14) du gaz de travail (16) agencés respectivement en amont et en aval dudit tronçon d'écoulement de sortie (10) par rapport à la direction du mouvement d'avance du gaz de travail lui-même vers ledit dispositif de mesure (5).

4. - Appareil (1) selon la revendication 3, **caractérisé par le fait que** les sections dudit premier tronçon de passage (13), dudit second tronçon de passage (14) et dudit tronçon d'écoulement de sortie (10) sont du type calibré.

5. - Appareil (1) selon la revendication 4, **caractérisé par le fait que** ledit premier tronçon de passage (13) a une section transversale pour le passage du gaz de travail (16) qui est supérieure ou égale à la section transversale pour le passage dudit tronçon d'écoulement de sortie (10).

6. - Appareil (1) selon une ou plusieurs des revendications 3 à 5, **caractérisé par le fait que** ledit second tronçon de passage (14) a une section transversale pour le passage du gaz de travail (16) qui est supérieure ou égale à la section transversale pour le passage dudit tronçon d'écoulement de sortie (10).

7. - Appareil (1) selon une ou plusieurs des revendications précédentes, **caractérisé par le fait que** ledit conduit de dérivation (9) est apte à être relié de manière amovible audit conduit de liaison (7).

8. - Appareil (1) selon une ou plusieurs des revendications 3 à 7, **caractérisé par le fait que** ledit conduit de dérivation (9) comprend au moins une paire de tronçons de liaison (11) qui sont reliés dans un mode à actionnement fluidique l'un à l'autre et audit tronçon d'écoulement de sortie (10) en un seul point de jonction (12), et **par le fait que** ledit premier tronçon de passage (13) est relié dans un mode à actionnement fluidique et amovible à ladite sortie (4) et à l'un desdits tronçons de liaison (11) et ledit second tronçon de passage (14) est relié dans un mode à actionnement fluidique et amovible à ladite entrée (6) et à l'autre desdits tronçons de liaison (11) .

9. - Appareil (1) selon la revendication 8, **caractérisé par le fait que** lesdits tronçons de liaison (11) sont agencés sensiblement convergents vers ledit tronçon d'écoulement de sortie (10).

10. - Appareil (1) selon l'une des revendications 8 ou 9, **caractérisé par le fait que** ledit conduit de dérivation (9) a une conformation en « Y ».

11. - Appareil (1) selon une ou plusieurs des revendications précédentes, **caractérisé par le fait que** ledit conduit de liaison (7) et ledit conduit de dérivation (9) sont réalisés en une seule pièce.
